# EUROPEAN PATENT SPECIFICATION

(11) **EP 3 515 346 B1**
(45) Date of publication and mention of the grant of the patent: **22.01.2020**
(21) Application number: 17768391.9
(22) Date of filing: 01.09.2017
(51) Int. Cl.: A61B 18/20, A61B 18/00

(54) **A CUTTING ASSEMBLY FOR A HAIR CUTTING DEVICE**
SCHNEIDVORRICHTUNG FÜR EINE HAARSCHNEIDEVORRICHTUNG
ENSEMBLE DE COUPE DESTINÉ À UN DISPOSITIF DE COUPE DE CHEVEUX

(30) Priority: 20.09.2016 EP 16189593
(43) Date of publication of application: 31.07.2019
(73) Proprietor: Koninklijke Philips N.V., 5656 AE Eindhoven (NL)
(72) Inventor: JOHNSON, Mark, Thomas, 5656 AE Eindhoven (NL); THUMMA, Kiran, Kumar, 5656 AE Eindhoven (NL); VERHAGEN, Rieko, 5656 AE Eindhoven (NL); MOESKOPS, Bastiaan, Wilhelmus, Maria, 5656 AE Eindhoven (NL); BOAMFA, Marius, Iosif, 5656 AE Eindhoven (NL)
(74) Representative: Uittenbroek, Arie Leendert
(86) International application number: PCT/EP2017/072032
(87) International publication number: WO 2018/054664

(56) References cited:
- EP-B1- 1 412 142
- WO-A1-2014/143670
- WO-A2-2009/090632
- US-A1- 2008 244 912
- US-A1- 2012 123 444
- US-A1- 2015 359 592

## Description

### FIELD OF THE INVENTION

The invention relates to a cutting assembly for use with a hair cutting device for cutting (e.g. shaving) hair on a body of a subject and, in particular, a cutting assembly having a portion that can be moved cyclically. The invention also relates to a hair cutting device comprising such a cutting assembly.

### BACKGROUND OF THE INVENTION

Shaving devices for cutting or shaving hair on a body of a subject typically make use of one or more blades that cut hairs as the device is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular, a laser light source is provided that is configured to generate laser light having a wavelength selected to target a predetermined chromophore to effectively cut a hair shaft. A fibre optic is located on a shaving portion of the device that is positioned to receive the laser light from the laser light source at a proximal end, conduct the laser light from the proximal end toward a distal end, and emit the light out of a cutting region of the fibre optic and toward hair when the cutting region is brought in contact with the hair.

### SUMMARY OF THE INVENTION

In order to cut or melt hair, sufficient optical energy needs to be delivered through a cutting element of the cutting device. While the cutting element is in contact with a hair, heat may be transferred from the hair into the cutting element. If the increase in temperature in the cutting element is too great, the cutting element may be damaged.

Thus, there exists a need for a cutting assembly having a cutting element which is less likely to break during the hair cutting process.

According to a first aspect, there is provided a cutting assembly for use in a hair cutting device, the cutting assembly comprising: an optical waveguide having a sidewall, wherein a portion of the sidewall forms a cutting face for contacting hair, wherein the optical waveguide is moveable between a first, retracted position and a second, extended position; and a movement mechanism for effecting cyclic movement of the optical waveguide between the retracted position and the extended position. By cyclically extending and retracting the optical waveguide, contact between the waveguide and the hair to be cut can be repeatedly made and broken. This prevents a significant increase in temperature of the optical waveguide and, thus, reduces the chance of the waveguide becoming damaged by the heat.

In some embodiments, the movement mechanism may be configured to move the optical waveguide linearly in a direction substantially perpendicular to an optical axis of the optical waveguide. In other embodiments, the movement mechanism may be configured to move the optical waveguide between the retracted position and the extended position along a non-linear path.

The movement mechanism may comprise a vibration mechanism for inducing a controlled vibration in the optical waveguide. By vibrating the optical waveguide, its movement between the retracted position and the extended position can be achieved rapidly, meaning contact with the hair can be made and broken numerous times before the hair is cut.

The movement mechanism may, in some embodiments, be configured to move the optical waveguide between the retracted position and the extended position at a defined speed or at a speed exceeding the defined speed. In other embodiments, the movement mechanism is configured to move the optical waveguide between the retracted position and the extended position at a speed exceeding a speed of movement of the cutting assembly over a surface containing hair intended to be cut.

The movement mechanism may comprise at least one of: a piezoelectric mechanism, a loudspeaker arrangement, a linear motor, and a capacitive micro-machined ultrasonic transducer, CMUT, device. Other mechanisms may also be used to effect movement of the waveguide.

In some embodiments, the optical waveguide comprises an optical fibre.

The cutting assembly may further comprise a spacing structure configured to support hair to be cut at a defined distance from the cutting face of the optical waveguide. In some embodiments, the spacing structure may comprise a length of wire oriented parallel to an optical axis of the optical waveguide and spaced from the optical waveguide. The spacing structure may be positioned such that, when the optical waveguide is in the second, extended position, the cutting face is adjacent to the spacing structure. Other spacing structures may alternatively be used, such as a structure which supports hair at a defined distance from the waveguide while also preventing the waveguide from touching the skin of the subject.

According to a second aspect, there is provided a hair cutting device for cutting hair on a body of a subject, the hair cutting device comprising: a light source for generating laser light at one or more specific wavelengths corresponding to wavelengths absorbed by one or more chromophores in hair; and a cutting assembly having a cutting element coupled to the light source to receive laser light, the cutting assembly comprising a cutting assembly as described above.

In some embodiments, the hair cutting device may further comprise a sensor for measuring the speed of movement of the hair cutting device over the body of the subject. The hair cutting device may also include a processor configured to vary the speed of movement of the optical waveguide between the retracted position and the extended position based on the measured speed of movement of the hair cutting device.

The movement mechanism may, in some embodiments, be configured to move the optical waveguide in a direction parallel to a direction of movement of the hair cutting device over the body of the subject.

Other advantageous features will become apparent from the following description.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to an embodiment of the invention;
Fig. 2 is a pair of schematic drawings showing different views of an exemplary hair cutting device according to embodiments of the invention;
Fig. 3 is a graph illustrating the refractive index of hair;
Fig. 4 is an illustration of a cutting assembly having a moveable optical waveguide according to embodiments of the invention;
Fig. 5 is an illustration of a cutting assembly having a moveable optical waveguide according to alternative embodiments of the invention;
Fig. 6 is an illustration of an example support structure according to embodiments of the invention;
Fig. 7 is an illustration of an example support structure according to embodiments of the invention; and
Fig. 8 is an illustration, in a plan view, of a cutting assembly according to embodiments of the invention.

### DETAILED DESCRIPTION OF THE EMBODIMENTS

As noted above, the present invention provides an improvement in the cutting ability and efficiency of a laser light-based shaving device, for example as described in WO 2014/143670. In particular, it has been recognised that by reducing the amount of time that a cutting element is in contact with a hair to be cut, the increase in temperature in the cutting element can be reduced, thereby reducing the chance of failure of the cutting element by damage caused by too much heat.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

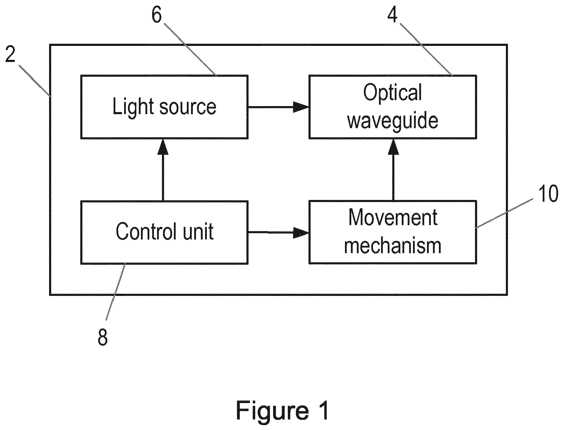

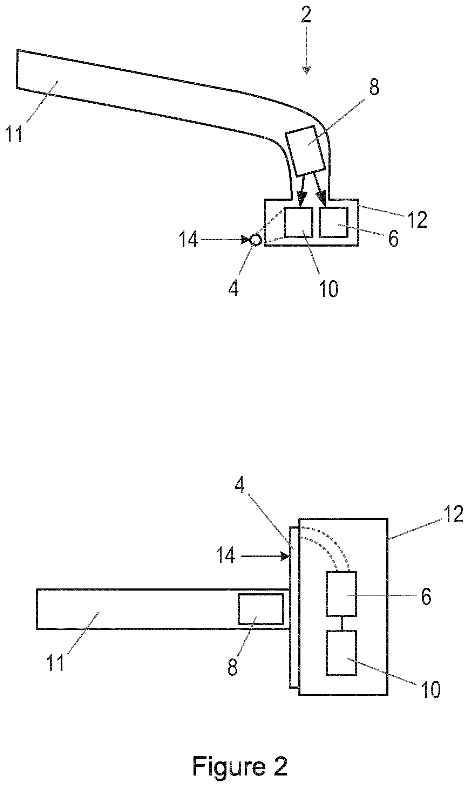

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. The cutting element 4 is an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the sidewall of the optical waveguide 4 (the sidewall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

A light source 6 is provided in the hair cutting device 2 that generates laser light at one or more specific wavelengths. The light source 6 is optically coupled to the optical waveguide 4. The laser light generated by the light source 6 is coupled into the optical waveguide 4 (and specifically coupled into an end of the optical waveguide 4 so that the laser light propagates through the optical waveguide 4).

The light source 6 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the light source 6 include, but are not limited to, melanin, keratin and water. Suitable wavelengths of laser light that can be used include, but are not limited to, wavelengths selected from the range 380 nm (nanometres) to 500 nm and 2500 nm to 3500 nm. Those skilled in the art will be aware of the wavelengths of light that are absorbed by these chromophores, and thus also the specific wavelengths of light that the light source 6 should generate for this purpose, and further details are not provided herein.

In some embodiments the light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources 6 can be provided that each generate laser light at a respective wavelength, and each light source 6 can be coupled to a respective optical waveguide 4 to provide multiple cutting elements 4 in the device 2.

The hair cutting device 2 also comprises a control unit 8 that controls the operation of the hair cutting device 2, and in particular is connected to the light source 6 to control the activation and deactivation of the light source 6 (and in some embodiments control the wavelength and/or intensity of the light generated by the light source 6). The control unit 8 may activate and deactivate the light source 6 in response to an input from a user of the hair cutting device 2. The control unit 8 can comprise one or more processors, processing units, multi-core processors or modules that are configured or programmed to control the hair cutting device 2.

The hair cutting device 2 also includes a movement mechanism 10 configured to move the optical waveguide 4 relative to the hair cutting device. Specifically, the movement mechanism is for effecting cyclic movement (i.e. movement that is repeated in cycles) of the optical waveguide between a first position and a second position, as is discussed in detail below. The movement mechanism 10 may be controlled by the control unit 8. In some embodiments, the hair cutting device 2 may also include a sensor (not shown) for measuring the speed of movement of the device 2 over a surface, as is discussed in greater detail below. The optical waveguide 4 and the movement mechanism 10 may be considered to form components of a cutting assembly which, in some embodiments, may be detachable from the hair cutting device 2, and may be enclosed in a separate unit or housing.

As noted above, Fig. 2 shows a hair cutting device 2 that is in the form of a handheld wet razor. Fig. 2 shows a side view and a bottom view of the razor 2. The razor 2 comprises a handle 11 for the subject (or other user of the device 2) to hold, and a head portion 12 that includes the cutting element 4 (optical waveguide/fibre) and the movement mechanism 10. As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. A light source 6 and the control unit 8 are shown as being incorporated into the head portion 12 and handle 11 respectively, but it will be appreciated that the positions of these components or the movement mechanism 10 in the hair cutting device 2 as shown in Fig. 2 is not limiting. Likewise it will be appreciated that the embodiment shown in Fig. 2 is merely an example, and the invention can be incorporated or used in any type of hair cutting device 2 that comprises an optical waveguide cutting element 4 as described herein. As noted above, the optical waveguide 4 and the movement mechanism 10 may form part of a cutting assembly which may itself form a part of, or be detachably connected to, the head portion 12.

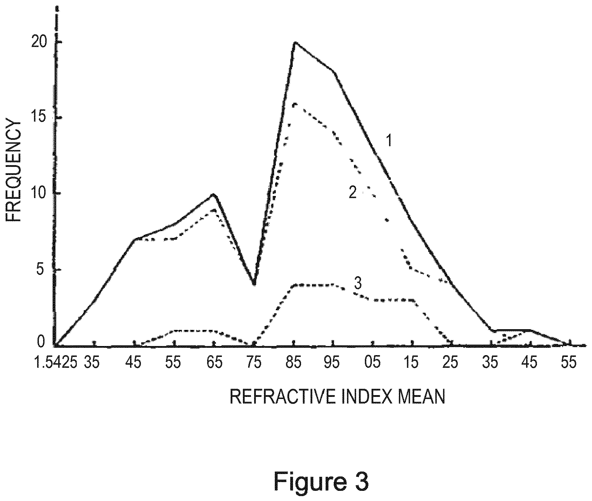

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light source 6 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 must have the same or a lower refractive index than hair at the point at which the hair contacts the optical waveguide 4. Thus, the optical waveguide 4 must have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair.

Thus, in some embodiments, the refractive index of the optical waveguide 4 at least at the cutting face 14 is equal to or lower than 1.56. More preferably the refractive index of the optical waveguide 4 at least at the cutting face 14 is equal to or lower than 1.55. Even more preferably, the refractive index of the optical waveguide 14 at least at the cutting face 14 is equal to or lower than 1.54, since this refractive index is below the refractive indices identified in Fig. 3.

In some embodiments, a lower bound for the refractive index of the optical waveguide 4 at the cutting face 14 can be 1.48, 1.51, 1.53 or 1.54.

A range of values from which the refractive index of the optical waveguide 4 is selected can be formed from any combination of the upper and lower refractive index bounds set out in the preceding paragraphs.

The optical waveguide/fibre 4 can be made from any suitable material or combination of materials. For example optical waveguides/fibres can be composed of or comprise silica, fluoride glass, phosphate glass, chalcogenide glass, crown glass (such as BK7) and/or crystals (such as sapphire or yttrium aluminium garnet (YAG)).

As noted above, while the optical waveguide/fibre 4 is in contact with a hair to be cut, the optical waveguide is caused to heat up significantly and, if the increase in temperature is too great, the optical waveguide may be damaged. The reason for the increase in temperature of the waveguide is that, as melting of the hair is initiated, the area of contact between the hair and the optical waveguide is increased and, as a result, more light from the waveguide is able to couple into the hair, causing a further increase in temperature. Therefore, it has been recognised that preventing prolonged periods of contact between the optical waveguide and strands of hair to be cut can prevent significant temperature increases in the optical waveguide and, therefore, can lower the likelihood that the optical waveguide will be damaged by high temperatures.

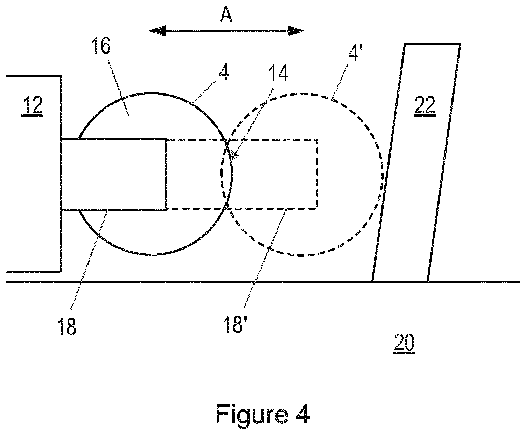

Fig. 4 illustrates an exemplary embodiment of part of a cutting assembly according to the invention. In Fig. 4, the cutting element (optical waveguide) 4 part of the hair cutting device 2 is shown, and the optical waveguide 4 is shown in side view (i.e. along an optical axis of the optical waveguide). The optical waveguide 4 has a sidewall, a portion of which forms the cutting face 14 for contacting hair. The optical waveguide 4 is moveable at least between two positions shown in Fig. 4: a first, retracted position, shown with a solid line and labelled 4, and a second, extended position, shown with a dashed line and labelled 4'. The cutting assembly also includes the movement mechanism (not shown in Fig. 4) as discussed herein for effecting cyclic movement of the optical waveguide 4 between the retracted position and the extended position. The cutting assembly, which includes the optical waveguide 4 and the movement mechanism 10 may be removably connectable to the hair cutting device 2 (for example to the head portion 12) such that the cutting assembly can be replaced when damaged, or replaced to suit different types of shaving or hair cutting, such as wet shaving, dry shaving or trimming. In some embodiments, the cutting element, or optical waveguide 4, may be separately removable and replaceable independently of the movement mechanism 10.

In Fig. 4, an optical waveguide 4 is shown that has a core 16. In this illustrated embodiment, the optical waveguide 4 does not include any cladding around the core 16. However, it will be appreciated that in some embodiments the optical waveguide 4 can comprise cladding around the core 16, although preferably no cladding is present along the cutting face 14 (and indeed, in some embodiments the cutting face 14 can correspond to those parts of the optical waveguide 4 where there is no cladding).

Part of the head portion 12 of the hair cutting device 2 is also shown in Fig. 4. The optical waveguide 4 may be connected to or mounted onto the head portion 12 of the hair cutting device 2 by a suitable mounting structure 18, or waveguide support, which may, in some embodiments, form part of the movement mechanism 10. The mounting structure 18 is also shown in two positions in Fig. 4: a first, retracted position, shown with a solid line and labelled 18, and a second, extended position, shown with a dashed line and labelled 18'. The mounting structure 18 may support the optical waveguide 4 at the ends of the waveguide, at positions spaced along the length of the waveguide, or along the entire length of the waveguide. The mounting structure 18 is configured to move with the optical waveguide 4. That is to say, when the optical waveguide is in its extended position (labelled 4'), the mounting structure is in its extended position (labelled 18') and when the optical waveguide is in its un-extended, or retracted position (labelled 4), the mounting structure is in its un-extended, or retracted position (labelled 18).

The portion of the sidewall of the core 16/optical waveguide 4 that is intended to contact hairs during use forms the cutting face 14. As described above, the refractive index of the core 16 is the same or lower than the refractive index of hair. The core 16 may have a uniform refractive index (i.e. the same refractive index throughout the core 16), or it may be a graded index fibre, which means that the refractive index decreases with increasing distance from the optical axis.

The optical waveguide 4 is shown positioned above the skin 20 of a subject from which a hair 22 extends, such that the cutting face 14 of the optical waveguide is able to contact, and thereby cut, the hair. In some embodiments, however, the optical waveguide 4 may be oriented or arranged such that it is in contact with the skin 20, and/or oriented such that the cutting face 14 of the waveguide is configured to contact the hair 22 at a higher or lower position, depending on the desired length of cut.

Movement of the optical waveguide 4 (and the mounting structure 18) between the retracted position and the extended position is effected by the movement mechanism 10. While, in Fig. 2, the movement mechanism 10 is shown located within the head portion 12 of the hair cutting device 2, the movement mechanism may be located elsewhere. For example, in some embodiments, the movement mechanism 10 may be coupled to the optical waveguide 4 or the mounting structure 18 (i.e. outside of the head portion 12) and, as noted above, the movement mechanism 10 may, in some embodiments, form part of the mounting structure 18.

The movement mechanism 10 is configured to effect cyclic movement of the optical waveguide between the retracted position and the extended position. The extended position of the optical waveguide (labelled 4' in Fig. 4), is intended to be the position in which the cutting face 14 can contact the hair 22 and effect melting or cutting thereof. In the retracted position (labelled 4 in Fig. 4), however, the optical waveguide is intended to be spaced away from (or retracted from) the hair 22 such that the cutting face 14 is not in contact with the hair. While the optical waveguide 4 is in contact with the hair 22, cutting or melting of the hair is initiated, and the increased temperature of the hair caused the waveguide to heat up. By retracting the optical waveguide 4 from the hair 22, the temperature of the optical waveguide can reduce. By allowing the temperature of the optical waveguide to reduce temporarily (or at least preventing prolonged contact with the hair which might result in a significant increase in the temperature of the waveguide), the chance that the optical waveguide will be damaged by heat is reduced.

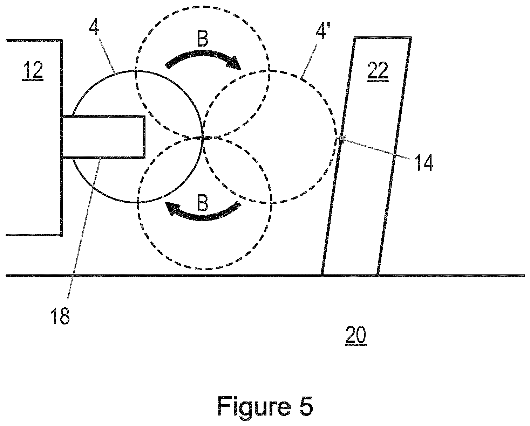

In the embodiment shown in Fig. 4, the movement mechanism 10 is configured to move the optical waveguide 4 linearly in a direction substantially perpendicular to an optical axis of the optical waveguide. The direction of movement of the optical waveguide 4 in the embodiment of Fig. 4 is shown by arrow A. An alternative embodiment is shown in Fig. 5, in which the movement mechanism 10 is configured to move the optical waveguide 4 between the retracted position and the extended position along a non-linear path. Fig. 5 shows the optical waveguide 4 coupled to the head portion 12 of the hair cutting device 2 by the mounting structure 18. In this embodiment, the optical waveguide 4 moves in a substantially circular path in the direction of arrows B, from the retracted position (labelled 4) to the extended position (labelled 4'), and back to the retracted position. In the extended position, the cutting face 14 of the optical waveguide 4 is able to contact the hair 22 to effect cutting or melting thereof. In this embodiment, the mounting structure 18 may also move along the non-linear path, pivot or extend as necessary to enable the optical waveguide 4 to move along the non-linear path. For clarity, the mounting structure 18 is shown only in the retracted position in Fig. 5, and not in the extended position.

The movement mechanism 10 may be configured to move the optical waveguide 4 in various ways and at different speeds, depending on the intended use of the hair cutting device 2. In some embodiments, the movement mechanism 10 may comprise a vibration mechanism for inducing a controlled vibration in the optical waveguide 4. The optical waveguide 4 may be configured to vibrate at a defined speed, and the distance that the waveguide moves during the vibration (i.e. the distance between the retracted position and the extended position) may be defined and controlled, for example, by the control unit 8. The movement mechanism 10 may comprise any suitable mechanism for effecting controlled movement of the optical waveguide 4 between the extended and retracted position. In some embodiments, the movement mechanism 10 may comprise at least one of: a piezoelectric mechanism, a loudspeaker arrangement, a linear motor, and a capacitive micro-machined ultrasonic transducer (CMUT) device.

As will be appreciated, if the movement mechanism 10 comprises a vibration mechanism, then the cyclic movement of the optical waveguide 4 between its extended and retracted positions is likely to be relatively fast. In some embodiments, the movement mechanism 10 may be configured (for example under control of the control unit 8) to move the optical waveguide 4 between the retracted position and the extended position at a speed exceeding a defined speed, or at a particular speed. For example, in some embodiments, the movement mechanism 10 may be configured to move the optical waveguide 4 between the retracted position and the extended position at a speed exceeding a speed of movement of the cutting assembly 4 (or the cutting device) over a surface containing hair intended to be cut. The defined speed may be selected based on an average, or typical, speed of movement of a hair cutting device 2 over the skin of a subject during a shaving or hair cutting activity. For example, a typical shaving speed (i.e. speed of movement of the hair cutting device 2 by a user over the skin) for facial shaving might be 10 centimetres per second (cm/s) or 20 cm/s, whereas a typical shaving speed for shaving other body parts, such as legs, might be 50 cm/s. Thus, the movement mechanism 10 may be configured to move the optical waveguide 4 between the retracted position and the extended position at a speed exceeding 10 cm/s, 20 cm/s or 50 cm/s, for example, depending on the intended use of the device 2. By moving the optical waveguide 4 between its retracted position and its extended position at a speed exceeding the speed at which the device 2 is moved over the skin, the waveguide will go through a cycle of contacting the hair 22 (when in the extended position) and being removed from (i.e. breaking contact with) the hair (when in the retracted position) at least once as the as the device is moved into proximity of the hair. If the optical waveguide 4 goes through more than one cycle for particular hair (that is, if the waveguide makes contact with the hair and breaks contact with the hair more than once), then the cutting ability of the cutting element may be improved.

In some embodiments, the speed of movement of the optical waveguide 4 between the retracted position and the extended position may be dynamic, such that the speed may be varied based on the speed of movement of the hair cutting device 2 over the skin. In such embodiments, the device 2 may include a speed sensor (not shown) for determining, in real time, the speed at which the device is moving over the skin 20 of a subject. The speed sensor may, in some embodiments, include an optical sensor, such as a laser sensor for determining the rate at which the device 2 is moved over the skin. In some embodiments, a sensor similar to that used in an optical computer mouse may be used. The movement mechanism 10 may be configured to move (or vibrate) the optical waveguide 4 in a cyclic manner at a speed based on the speed determined by the speed sensor. For example, the movement mechanism 10 may be configured to move (or vibrate) the optical waveguide 4 at a speed equal to or exceeding the speed determined by the speed sensor.

It is estimated that, using a light source 6 having an output power of 1 Watt, the total time needed to fully cut a hair (in other words, the cutting time) using a cutting element 4 as described herein is between around 1 millisecond (ms) to 10 ms. If a lower-powered light source is used, then the required cutting time may be longer, and if a higher-powered light source is used, then the required cutting time may be shorter. Rather than leaving the optical waveguide 4 in contact with the hair for the entire cutting time (which would likely cause a large increase in temperature in the waveguide), the optical waveguide 4 is touched against the hair (i.e. while in the extended position) for a short time to initiate the cutting or melting of the hair, then moved away from the hair (i.e. while in the retracted position) for a short time. This prevents a significant increase of temperature in the waveguide. The optical waveguide is then moved back into contact with the hair for a short time (in the extended position) to effect further cutting of the hair. In this way, the duration of contact between the optical waveguide 4 and the hair (i.e. the contact time) is less than the cutting time. Suitable contact times may be around 0.1 ms to 5 ms. Such contact times can be achieved by moving (or vibrating) the optical waveguide through the movement cycle (moving from the retracted position into the extended position and back to the retracted position) at a frequency of around 200 Hz to 100 kHz.

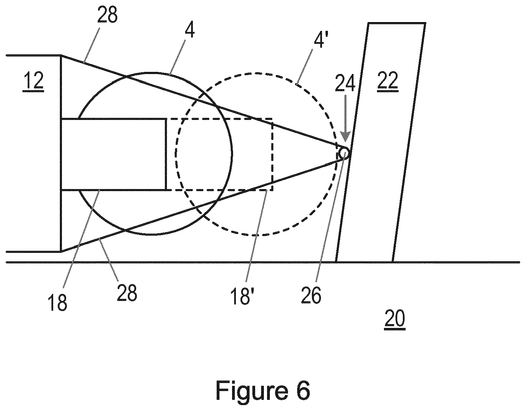

Figs. 6, 7 and 8 show parts of a cutting assembly constructed according to alternative embodiments of the invention. The optical waveguide 4, the mounting structure 18 and the manner in which those features are connected to the head portion 12 of the hair cutting device 2 in each of the embodiments of Figs. 6, 7 and 8, are similar to those shown in Fig. 4. However, in each of Figs. 6, 7 and 8 the cutting assembly further includes a spacing structure 24. The spacing structure 24 is configured to support hair 22 to be cut at a defined distance from the cutting face 14 of the optical waveguide 4. The spacing structure 24 includes a cross member 26 extending substantially parallel to the optical axis of the optical waveguide 4, and along at least part of the length of the optical waveguide. The cross member 26 is connected to the hair cutting device by one or more support members 28, which may support the cross member at its ends, and/or at one or more locations along its length. The one or more support members 28 may be connected to the support structures 18, the head portion 12, or elsewhere on the hair cutting device 2.

The cross member 26 of Fig. 6 is positioned in front of the cutting face 14 such that, when the optical waveguide 4 is in its second, extended position, the cutting face is adjacent to the cross member of the spacing structure 24.

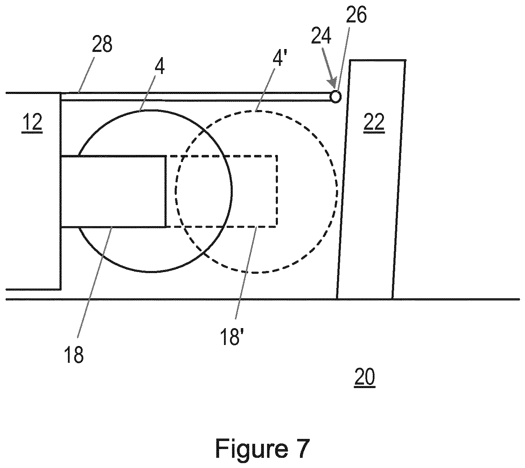

In the embodiment shown in Fig. 7, the spacing structure 24 is positioned at or near to a top edge of the head portion 12, such that the cross member 26 engages the hair 22 above the area where the cutting face 14 engages the hair when the optical waveguide 4 is in the extended position. It will be appreciated that the spacing structure 24 may be configured and arranged in numerous other ways that enable the cross member 26 to engage the hair 22 and support the hair at a defined distance from the cutting face 14 of the optical waveguide.

In some embodiments, the cross member 26 of the spacing structure 24 comprises a length of wire oriented parallel to an optical axis of the optical waveguide 4 and spaced from the optical waveguide. In other embodiments, the spacing structure may comprise a bar or rod, or a length of material stretched between support members 28.

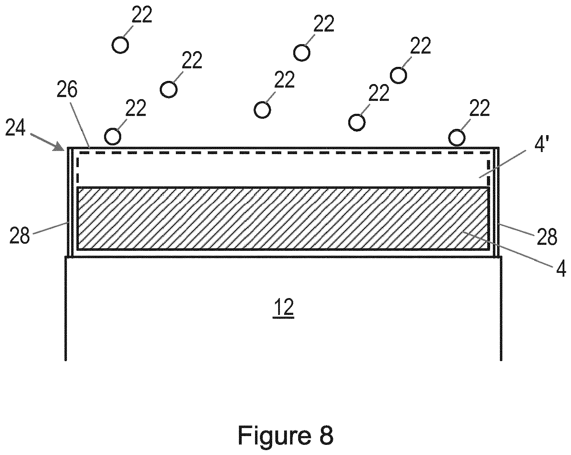

Fig. 8 shows a plan view of the arrangements shown in Figs. 6 and 7. As the hair cutting device 2 is moved towards the hairs 22, the cross member 26 of the spacing structure 24 contacts each hair, and supports the hair at a minimum defined distance from the cutting face 14 of the optical waveguide 4. The use of the spacing structure 24 in the cutting assembly makes the cutting performance substantially independent of the speed of movement of the device 2 over the skin 20 as the spacing structure ensures that the hair is held at substantially a fixed position relative to the optical waveguide for the time needed to cut the hair. In some embodiments, the spacing structure 24 may be configured such that the distance between the retracted position and the extended position of the optical waveguide 4 is relatively small (relative to a cutting assembly which does not include the support structure) and, therefore the distance over which the optical waveguide is to be moved between the retracted position and the extended position can be reduced.

As noted above, the cutting assembly may form part of the hair cutting device 2. The hair cutting device 2 may comprise the light source 6 for generating laser light at one or more specific wavelengths corresponding to wavelengths absorbed by one or more chromophores in hair; and a cutting assembly having a cutting element coupled to the light source to receive laser light, the cutting assembly comprising a cutting assembly as described herein. As noted above, the hair cutting device 2 may include a sensor for measuring the speed of movement of the hair cutting device over the body of the subject, and may include a processor configured to vary the speed of movement of the optical waveguide 4 based on the measured speed of movement of the hair cutting device. The processor may comprise the control unit 8. The movement mechanism 10 may be configured to move the optical waveguide 4 in a direction parallel to a direction of movement of the hair cutting device over the body of the subject, and such movement by the movement mechanism may be in a linear or a non-linear manner.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A cutting assembly for use in a hair cutting device, the cutting assembly comprising:
an optical waveguide having a sidewall corresponding to a long edge of said optical waveguide,
wherein a portion of the sidewall forms a cutting face for contacting hair,
wherein the optical waveguide is moveable between a first, retracted position in which the cutting face is arranged not to contact a hair during hair cutting and a second, extended position in which the cutting face is arranged to contact a hair during hair cutting; and
a movement mechanism for effecting cyclic movement of the optical waveguide between the retracted position and the extended position.

2. A cutting assembly according to claim 1, wherein the movement mechanism is configured to move the optical waveguide linearly in a direction substantially perpendicular to an optical axis of the optical waveguide.

3. A cutting assembly according to claim 1, wherein the movement mechanism is configured to move the optical waveguide between the retracted position and the extended position along a non-linear path.

4. A cutting assembly according to claim 1 or claim 2, wherein the movement mechanism comprises a vibration mechanism for inducing a controlled vibration in the optical waveguide.

5. A cutting assembly according to any of the preceding claims, wherein the movement mechanism is configured to move the optical waveguide between the retracted position and the extended position at a defined speed or a speed exceeding the defined speed.

6. A cutting assembly according to claim 5, wherein the defined speed is a speed of movement of the cutting assembly over a surface containing hair intended to be cut.

7. A cutting assembly according to any of the preceding claims, wherein the movement mechanism comprises at least one of: a piezoelectric mechanism, a loudspeaker arrangement, a linear motor, and a capacitive micro-machined ultrasonic transducer, CMUT, device.

8. A cutting assembly according to any of the preceding claims, wherein the optical waveguide comprises an optical fibre.

9. A cutting assembly according to any of the preceding claims, further comprising:
a spacing structure configured to support hair to be cut at a defined distance from the cutting face of the optical waveguide.

10. A cutting assembly according to claim 9, wherein the spacing structure comprises a length of wire oriented parallel to an optical axis of the optical waveguide and spaced from the optical waveguide.

11. A cutting assembly according to claim 9 or claim 10, wherein the spacing structure is positioned such that, when the optical waveguide is in the second, extended position, the cutting face is adjacent to the spacing structure.

12. A hair cutting device for cutting hair on a body of a subject, the hair cutting device comprising:
a light source for generating laser light at one or more specific wavelengths corresponding to wavelengths absorbed by one or more chromophores in hair; and
a cutting assembly having a cutting element coupled to the light source to receive laser light, the cutting assembly comprising a cutting assembly according to any of the preceding claims.

13. A hair cutting device according to claim 12, further comprising:
a sensor for measuring the speed of movement of the hair cutting device over the body of the subject.

14. A hair cutting device according to claim 13, further comprising:
a processor configured to vary the speed of movement of the optical waveguide between the retracted position and the extended position based on the measured speed of movement of the hair cutting device.

15. A hair cutting device according to any of claims 12 to 14, wherein the movement mechanism is configured to move the optical waveguide in a direction parallel to a direction of movement of the hair cutting device over the body of the subject.

## Patentansprüche

1. Schneidekonstruktion zur Verwendung bei einer Haarschneidevorrichtung, wobei die Schneidekonstruktion Folgendes umfasst:
einen optischer Wellenleiter, der eine Seitenwand aufweist, die einer langen Kante des optischen Wellenleiters entspricht,
wobei ein Abschnitt der Seitenwand eine Schneidefläche zum In-Kontakt-Treten mit Haar bildet,
wobei der optische Wellenleiter zwischen einer ersten, zurückgezogenen Position, in der die Schneidefläche angeordnet ist, um während des Haareschneidens nicht mit Haar in Kontakt zu treten, und einer zweiten, ausgezogenen Position, in der die Schneidefläche angeordnet ist, um während des Haareschneidens mit Haar in Kontakt zu treten, bewegbar ist; und
einen Bewegungsmechanismus zum Bewirken einer zyklischen Bewegung des optischen Wellenleiters zwischen der zurückgezogenen Position und der ausgezogenen Position.

2. Schneidekonstruktion nach Anspruch 1, wobei der Bewegungsmechanismus konfiguriert ist, um den optischen Wellenleiter linear in eine Richtung zu bewegen, die im Wesentlichen senkrecht zu einer optischen Achse des optischen Wellenleiters ist.

3. Schneidekonstruktion nach Anspruch 1, wobei der Bewegungsmechanismus konfiguriert ist, um den optischen Wellenleiter zwischen der zurückgezogenen Position und der ausgezogenen Position entlang eines nichtlinearen Weges zu bewegen.

4. Schneidekonstruktion nach Anspruch 1 oder Anspruch 2, wobei der Bewegungsmechanismus einen Schwingungsmechanismus zum Auslösen einer gesteuerten Schwingung in dem optischen Wellenleiter umfasst.

5. Schneidekonstruktion nach einem der vorstehenden Ansprüche, wobei der Bewegungsmechanismus konfiguriert ist, um den optischen Wellenleiter zwischen der zurückgezogenen Position und der ausgezogenen Position mit einer definierten Geschwindigkeit oder einer Geschwindigkeit, die die definierte Geschwindigkeit übersteigt, zu bewegen.

6. Schneidekonstruktion nach Anspruch 5, wobei die definierte Geschwindigkeit eine Geschwindigkeit der Bewegung der Schneidekonstruktion über eine Oberfläche, die Haar enthält, das geschnitten werden soll, ist.

7. Schneidekonstruktion nach einem der vorstehenden Ansprüche, wobei der Bewegungsmechanismus mindestens einen von folgenden umfasst: einen piezoelektrischen Mechanismus, eine Lautsprecheranordnung, einen linearen Motor und eine kapazitiver mikrobearbeiteter Ultraschallwandler, CMUT, -Vorrichtung.

8. Schneidekonstruktion nach einem der vorstehenden Ansprüche, wobei der optische Wellenleiter einen Lichtwellenleiter umfasst.

9. Schneidekonstruktion nach einem der vorstehenden Ansprüche, weiter umfassend:
eine Beabstandungsstruktur, die konfiguriert ist, um Haar, das geschnitten werden soll, in einem definierten Abstand von der Schneidefläche des optischen Wellenleiters zu tragen.

10. Schneidekonstruktion nach Anspruch 9, wobei die Beabstandungsstruktur eine Drahtlänge umfasst, die zu einer optischen Achse des optischen Wellenleiters parallel und von dem optischen Wellenleiter beabstandet ist.

11. Schneidekonstruktion nach Anspruch 9 oder Anspruch 10, wobei die Beabstandungsstruktur so positioniert ist, dass, wenn sich der Wellenleiter in der zweiten, ausgezogenen Position befindet, die Schneidefläche der Beabstandungsstruktur benachbart ist.

12. Haarschneidevorrichtung zum Schneiden von Haar auf einem Körper eines Subjekts, wobei die Haarschneidevorrichtung Folgendes umfasst:
eine Lichtquelle zum Erzeugen von Laserlicht bei einer oder mehreren spezifischen Wellenlängen, die Wellenlängen entsprechen, die von einem oder mehreren Chromophoren im Haar absorbiert werden; und
eine Schneidekonstruktion, die ein Schneideelement aufweist, das mit der Lichtquelle gekoppelt ist, um Laserlicht zu empfangen, wobei die Schneidekonstruktion eine Schneidekonstruktion nach einem der vorstehenden Ansprüche umfasst.

13. Haarschneidevorrichtung nach Anspruch 12, weiter umfassend:
einen Sensor zum Messen der Geschwindigkeit der Bewegung der Haarschneidevorrichtung über den Körper des Subjekts.

14. Haarschneidevorrichtung nach Anspruch 13, weiter umfassend:
einen Prozessor, der konfiguriert ist, um die Geschwindigkeit der Bewegung des optischen Wellenleiters zwischen der zurückgezogenen Position und der ausgezogenen Position basierend auf der gemessenen Bewegungsgeschwindigkeit der Haarschneidevorrichtung zu variieren.

15. Haarschneidevorrichtung nach einem der Ansprüche 12 bis 14, wobei der Bewegungsmechanismus konfiguriert ist, um den optischen Wellenleiter in eine Richtung parallel zu einer Bewegungsrichtung der Haarschneidevorrichtung über den Körper des Subjekts zu bewegen.

## Revendications

1. Ensemble de coupe à utiliser dans un dispositif de coupe de poils, l'ensemble de coupe comprenant :
un guide d'ondes optique présentant une paroi latérale correspondant à un bord long dudit guide d'ondes optique,
dans lequel une partie de la paroi latérale forme une face de coupe pour être en contact avec des poils,
dans lequel le guide d'ondes optique est mobile entre une première position rangée dans laquelle la face de coupe est agencée pour ne pas être en contact avec des poids pendant la coupe de poils et une seconde position déployée dans laquelle la face de coupe est agencée pour être en contact avec des poils pendant la coupe de poils ; et
un mécanisme de mouvement pour effectuer un mouvement cyclique du guide d'ondes optique entre la position rangée et la position déployée.

2. Ensemble de coupe selon la revendication 1, dans lequel le mécanisme de mouvement est configuré pour déplacer le guide d'ondes optique linéairement dans une direction sensiblement perpendiculaire à un axe optique du guide d'ondes optique.

3. Ensemble de coupe selon la revendication 1, dans lequel le mécanisme de mouvement est configuré pour déplacer le guide d'ondes optique entre la position rangée et la position déployée le long d'un trajet non linéaire.

4. Ensemble de coupe selon la revendication 1 ou la revendication 2, dans lequel le mécanisme de mouvement comprend un mécanisme de vibration pour induire une vibration commandée dans le guide d'ondes optique.

5. Ensemble de coupe selon l'une quelconque des revendications précédentes, dans lequel le mécanisme de mouvement est configuré pour déplacer le guide d'ondes optique entre la position rangée et la position déployée à une vitesse définie ou à une vitesse dépassant la vitesse définie.

6. Ensemble de coupe selon la revendication 5, dans lequel la vitesse définie est une vitesse de mouvement de l'ensemble de coupe sur une surface contenant des poils destinés à être coupés.

7. Ensemble de coupe selon l'une quelconque des revendications précédentes, dans lequel le mécanisme de mouvement comprend au moins un de : un mécanisme piézoélectrique, un agencement de haut-parleurs, un moteur linéaire et un dispositif transducteur ultrasonique capacitif micro-usiné, CMUT.

8. Ensemble de coupe selon l'une quelconque des revendications précédentes, dans lequel le guide d'ondes optique comprend une fibre optique.

9. Ensemble de coupe selon l'une quelconque des revendications précédentes, comprenant en outre :
une structure d'espacement configurée pour supporter des poils à couper à une distance définie de la face de coupe du guide d'ondes optique.

10. Ensemble de coupe selon la revendication 9, dans lequel la structure d'espacement comprend une longueur d'un fil orientée parallèlement à un axe optique du guide d'ondes optique et espacée du guide d'ondes optique.

11. Ensemble de coupe selon la revendication 9 ou la revendication 10, dans lequel la structure d'espacement est positionnée de telle sorte que, lorsque le guide d'ondes optique est dans la seconde position déployée, la face de coupe est adjacente à la structure d'espacement.

12. Dispositif de coupe de poils pour couper des poils sur un corps d'un sujet, le dispositif de coupe de poils comprenant :
une source de lumière pour générer une lumière laser à une ou plusieurs longueurs d'onde spécifiques correspondant à des longueurs d'onde absorbées par un ou plusieurs chromophores dans les poils ; et
un ensemble de coupe ayant un élément de coupe couplé à la source de lumière pour recevoir la lumière laser, l'ensemble de coupe comprenant un ensemble de coupe selon l'une quelconque des revendications précédentes.

13. Dispositif de coupe de poils selon la revendication 12, comprenant en outre :
un capteur pour mesurer la vitesse de mouvement du dispositif de coupe de poils sur le corps du sujet.

14. Dispositif de coupe de poils selon la revendication 13, comprenant en outre :
un processeur configuré pour faire varier la vitesse de mouvement du guide d'ondes optique entre la position rangée et la position déployée sur la base de la vitesse de mouvement mesurée du dispositif de coupe de poils.

15. Dispositif de coupe de poils selon l'une quelconque des revendication 12 à 14, dans lequel le mécanisme de mouvement est configuré pour déplacer le guide d'ondes optique dans une direction parallèle à une direction de mouvement du dispositif de coupe de poils sur le corps du sujet.
